# EUROPEAN PATENT APPLICATION

(11) **EP 2 980 090 A1**
(43) Date of publication of application: **03.02.2016**
(21) Application number: 14774738.0
(22) Date of filing: 28.03.2014
(51) Int. Cl.: C07D 493/08, A61K 31/437, A61P 7/02

(54) **METHOD FOR PRODUCING (1S,4S,5S)-4-BROMO-6-OXABICYCLO[3.2.1]OCTAN-7-ONE**

(30) Priority: 29.03.2013 JP 2013072896
(71) Applicant: Daiichi Sankyo Co., Ltd., Chuo-ku Tokyo 103-8426 (JP)
(72) Inventor: TAKAYANAGI, Yoshihiro, Hiratsuka-shi Kanagawa 254-0014 (JP); FURUYA, Yukito, Hiratsuka-shi Kanagawa 254-0014 (JP); NAKAMURA, Yoshitaka, Hiratsuka-shi Kanagawa 254-0014 (JP)
(74) Representative: Fairbairn, Angus Chisholm
(86) International application number: PCT/JP2014/059101
(87) International publication number: WO 2014/157612

(57) **Abstract**

It is an object of the present invention to provide a method for efficiently producing (1S,4S,5S)-4-bromo-6-oxabicyclo[3.2.1]octan-7-one (1), which is important as an intermediate compound for the production of an FXa-inhibiting compound. A method for producing (1S,4S,5S)-4-bromo-6-oxabicyclo[3.2.1]octan-7-one (1), which comprises treating an (R)-α-phenylethylamine salt of (S)-3-cyclohexene-1-carboxylic acid with 1,3-dibromo-5,5-dimethylhydantoin or N-bromosuccinimide in a solvent.

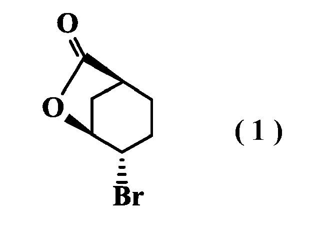

## Description

### Technical Field

The present invention relates to a method for producing an intermediate compound for the production of a compound that exhibits an inhibitory action on activated blood coagulation factor X (FXa) and that is useful as a preventive and/or therapeutic agent for thrombotic disease.

### Background Art

As compounds that exhibit an inhibitory action on activated blood coagulation factor X (hereinafter sometimes referred to as FXa) and are useful as preventive and/or therapeutic agents for thrombotic disease, the following compounds, for example, are known: N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (X): 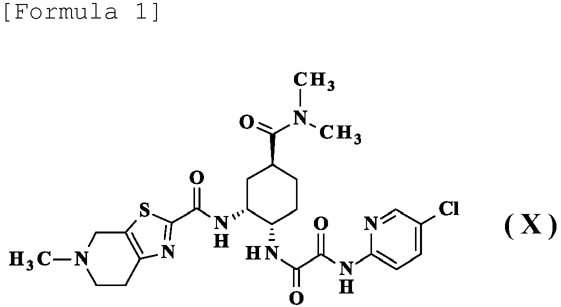 or a salt thereof, or their hydrate, and also, for example, a p-toluenesulfonate monohydrate of compound (X) represented by the following formula (X-a): 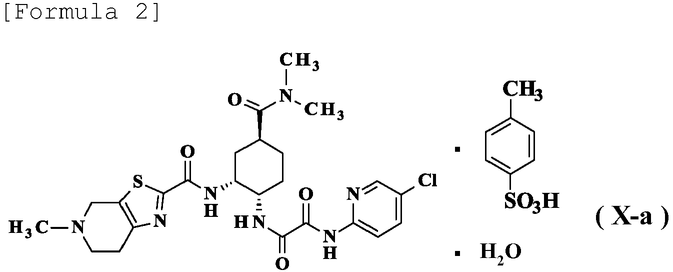 (see, for example, Patent Literatures 2 to 5).

As an intermediate compound for the production of these FXa-inhibiting compounds, (1S,4S,5S)-4-iodo-6-oxabicyclo[3.2.1]octan-7-one [hereinafter sometimes referred to as compound (1-a)] represented by the following formula (1-a) : 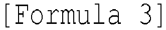 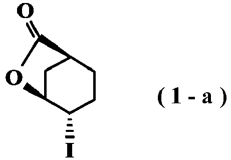 has been disclosed (see, for example, Patent Literatures 1 to 5 and Non-patent Literature 1). Moreover, it has been reported that compound (1-a), or a bromo derivative thereof that is (1S,4S,5S)-4-bromo-6-oxabicyclo[3.2.1]octan-7-one represented by the following formula (1) : 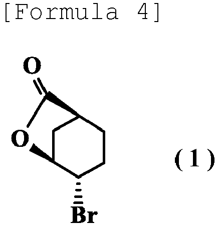 can be synthesized by a halolactonization (bromolactonization) reaction of optically active (S)-3-cyclohexene-1-carboxylic acid (2-a) (see, for example, Non-patent Literature 2) represented by the following formula (2-a): 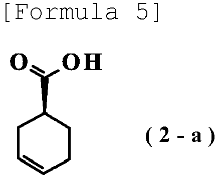 (see, for example, Non-patent Literatures 3 to 7).

However, a method for producing compound (1) from an optically active salt compound (2-b), which is obtained by fractional recrystallization of a salt compound obtained from 3-cyclohexene-1-carboxylic acid (2) as a racemic form and an optically active amine (3), as shown in the following scheme, without neutralizing compound (2-b) to convert it to a free acid, is not yet known.

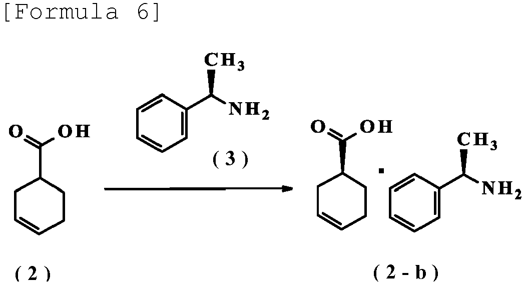

### Citation List

### Patent Literature

Patent Literature 1: International Publication No. WO 2003/000657
Patent Literature 2: International Publication No. WO 2003/000680
Patent Literature 3: International Publication No. WO 2003/016302
Patent Literature 4: International Publication No. WO 2004/058715
Patent Literature 5: International Publication No. WO 2001/074774

### Non-patent Literature

Non-patent Literature 1: K. Miyashita et al., Tetrahedron, 67(11), 2011, 2044-2050.
Non-patent Literature 2: H. M. Schwartz et al., J. Am. Chem. Soc., 100, 5199-5203, 1978.
Non-patent Literature 3: F. Chen et al., Tetrahedron Letters, 51(6), (2010), 3433-3435.
Non-patent Literature 4: M. Chini et al., Tetrahedron, 48(3), 539-544, 1992.
Non-patent Literature 5: Y. Fujimoto et al., Heterocycles, 23 (8), 2035-2039, 1985.
Non-patent Literature 6: C. Iwata et al., Heterocycles, 31(6), 987-991, 1990.
Non-patent Literature 7: J. C. S., Perkin Trans. 1, (1994), 7, 847-51.

### Summary of Invention

### Technical Problem

It is an object of the present invention to provide a method for efficiently producing (1S,4S,5S)-4-bromo-6-oxabicyclo[3.2.1]octan-7-one (1), which is important as an intermediate compound for the production of an FXa-inhibiting compound, from an (R)-α-phenylethylamine salt of (S)-3-cyclohexene-1-carboxylic acid.

### Solution to Problem

As a result of intensive studies directed towards achieving the aforementioned object, the present inventors have found that high-purity (1S,4S,5S)-4-bromo-6-oxabicyclo[3.2.1]octan-7-one (1) can be efficiently obtained by treating an (R)-α-phenylethylamine salt of (S)-3-cyclohexene-1-carboxylic acid (2-b), in the form of the salt without converting it to the free acid, with 1,3-dibromo-5,5-dimethylhydantoin (4) or N-bromosuccinimide (5) in a solvent, thereby completing the present invention.

### Advantageous Effect of Invention

According to the present invention, a method for efficiently producing (1S,4S,5S)-4-bromo-6-oxabicyclo[3.2.1]octan-7-one (1), which is important as an intermediate compound for the production of an FXa-inhibiting compound, is provided.

### Description of Embodiments

Specifically, the present invention provides the following [1] to [5].
[1] A method for producing a compound represented by the following formula (1) : 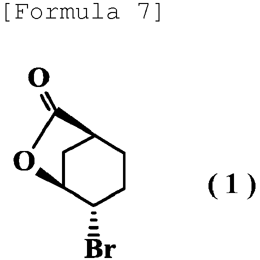 wherein the method comprises treating an (R)-α-phenylethylamine salt of (S)-3-cyclohexene-1-carboxylic acid represented by the following formula (2-b): 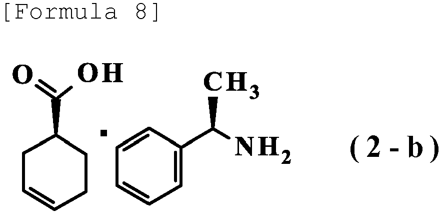 with 1,3-dibromo-5,5-dimethylhydantoin represented by the following formula (4): 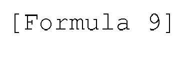 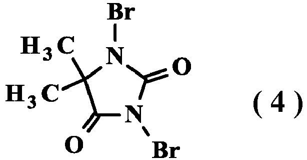 or with N-bromosuccinimide represented by the following formula (5): 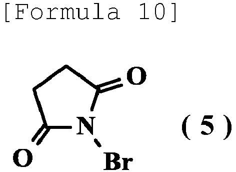 in a solvent.
[2] A method for producing a compound represented by the following formula (1) : 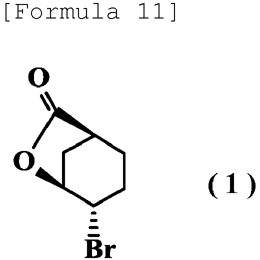 wherein the method comprises treating 3-cyclohexene-1-carboxylic acid represented by the following formula (2) : 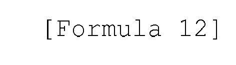 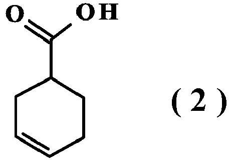 with (R)-α-phenylethylamine represented by the following formula (3): 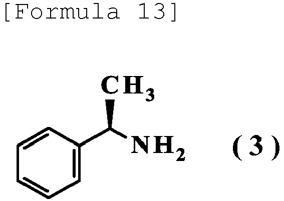 to obtain an (R)-α-phenylethylamine salt of (S)-3-cyclohexene-1-carboxylic acid represented by the following formula (2-b): 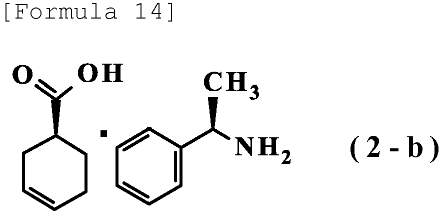 and then treating the (R)-α-phenylethylamine salt of (S)-3-cyclohexene-1-carboxylic acid with 1,3-dibromo-5,5-dimethylhydantoin represented by the following formula (4) : 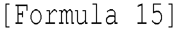 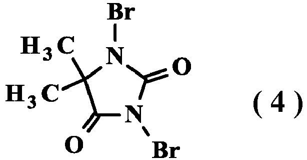 or with N-bromosuccinimide represented by the following formula (5): 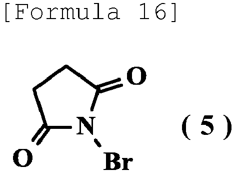 in a solvent.
[3] The production method according to [1] or [2], wherein the solvent is acetonitrile.
[4] A method for producing a compound represented by the following formula (11a) : 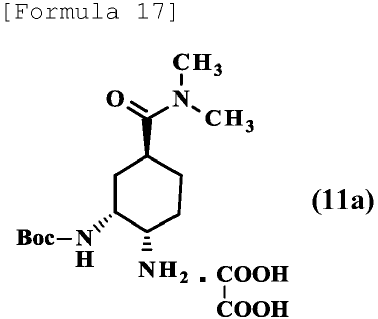 wherein Boc represents a tert-butoxycarbonyl group;
   wherein (1S,4S,5S)-4-bromo-6-oxabicyclo[3.2.1]octan-7-one (1) represented by the formula (1), which is produced by the production method according to [1], is used, and the method comprises the following steps (a) and (b):
   step (a): treating the (1S,4S,5S)-4-bromo-6-oxabicyclo[3.2.1]octan-7-one (1) with a dimethylamine aqueous solution, then treating the resulting compound with an ammonia aqueous solution, then treating the resulting compound with di-tert-butyl dicarbonate, and then treating the resulting compound with methanesulfonyl chloride to produce methanesulfonic acid (1R,2R,4S)-2-tert-butoxycarbonylamino-4-dimethylcarbamoyl-cyclohexyl ester; and
   step (b): treating the methanesulfonic acid (1R,2R,4S)-2-tert-butoxycarbonylamino-4-dimethylcarbamoyl-cyclohexyl ester with sodium azide, then hydrogenating the resulting compound in the presence of palladium-carbon and ammonium formate, and then treating the resulting compound with oxalic acid to produce tert-butyl {(1R,2S,5S)-2-amino-5-[(dimethylamino)carbonyl]cyclohexyl}carbamate oxalate.
[5] A method for producing N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide p-toluenesulfonate monohydrate represented by the following formula (X-a): 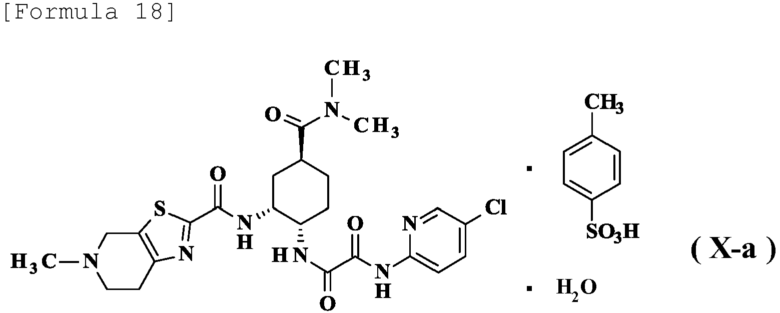 wherein the (1S,4S,5S)-4-bromo-6-oxabicyclo[3.2.1]octan-7-one (1) represented by the formula (1), which is produced by the production method according to [1], is used, and the method comprises the following steps (a) to (e):
   step (a): treating the (1S,4S,5S)-4-bromo-6-oxabicyclo[3.2.1]octan-7-one (1) with a dimethylamine aqueous solution, then treating the resulting compound with an ammonia aqueous solution, then treating the resulting compound with di-tert-butyl dicarbonate, and then treating the resulting compound with methanesulfonyl chloride to produce methanesulfonic acid (1R,2R,4S)-2-tert-butoxycarbonylamino-4-dimethylcarbamoyl-cyclohexyl ester;
   step (b): treating the methanesulfonic acid (1R,2R,4S)-2-tert-butoxycarbonylamino-4-dimethylcarbamoyl-cyclohexyl ester with sodium azide, then hydrogenating the resulting compound in the presence of palladium-carbon and ammonium formate, and then treating the resulting compound with oxalic acid to produce tert-butyl {(1R,2S,5S)-2-amino-5-[(dimethylamino)carbonyl]cyclohexyl}carbamate oxalate;
   step (c): treating the tert-butyl {(1R,2S,5S)-2-amino-5-[(dimethylamino)carbonyl]cyclohexyl}carbamate oxalate with ethyl [5-chloropyridin-2-yl]amino](oxo)acetate hydrochloride in the presence of triethylamine to produce tert-butyl [[1R,2S,5S]-2-({[(5-chloropyridin-2-yl)amino](oxo)acetyl}amino)-5-(dimethylaminocarbonyl)cyclohexyl]carbamate;
   step (d): treating the tert-butyl [(1R,2S,5S)-2-({[(5-chloropyridin-2-yl)amino](oxo)acetyl}amino)-5-(dimethylaminocarbonyl)cyclohexyl]carbamate with methanesulfonic acid, and then treating the resulting compound with 5-methyl-4,5,6,7-tetrahydro[1,3]thiazolo[5,4-c]pyridine-2-carboxylate hydrochloride to produce N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide [edoxaban]; and
   step (e): treating the N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide [edoxaban] with p-toluenesulfonic acid in aqueous ethanol to produce N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide p-toluenesulfonate monohydrate.

Hereinafter, the present invention will be described in detail.

In the present description, "C1-C6 alkyl" means a linear or branched alkyl group containing 1 to 6 carbon atoms. Examples of the C1-C6 alkyl in the present description include methyl, ethyl, propyl, isopropyl, and n-butyl.

Examples of "C1-C6 alkyl alcohol" in the present description include methanol, ethanol, propanol, isopropyl alcohol, and n-butanol.

In the present description, "aqueous solvent" means a mixed solvent of water and an organic solvent. The mixing of water and an organic solvent may be carried out before the reaction or in mid-course of the reaction. The timing of the mixing of the water and the organic solvent is not particularly limited, as long as it is in an environment in which the water and the organic solvent act as solvents.

In the present description, "equivalent" means a stoichiometric molar equivalent, unless otherwise specified.

In the present invention, N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (X): 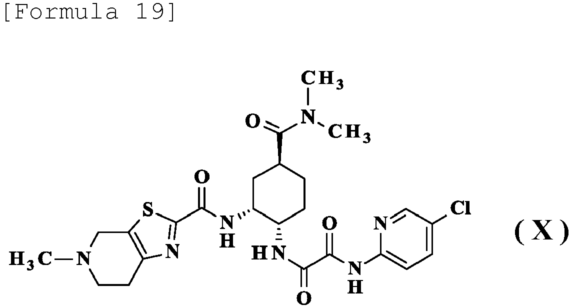 is the free form (free base) of compound (X-a) that is a p-toluenesulfonate monohydrate, and this compound has been registered with the World Health Organization (WHO) as N-(5-chloropyridin-2-yl)-N'-[(1S,2R,4S)-4-(N,N-dimethylcarbamoyl)-2-(5-methyl-4,5,6,7-tetrahydro[1,3]thiazolo[5,4-c]pyridine-2-carboxamido)cyclohexyl]oxamide, International Nonproprietary Name (INN): edoxaban.

The above described compound (X) may be a pharmacologically acceptable salt thereof, or may also be their hydrate. As such compound (X), N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (X-a): 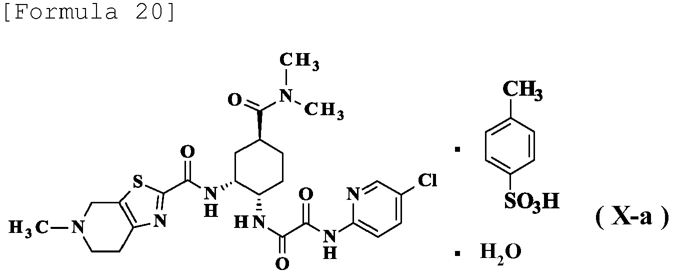 is preferable.

The production method of the present invention will be described in detail below.

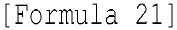

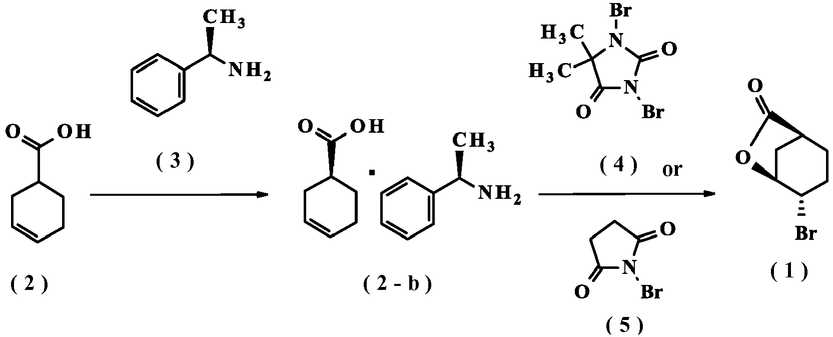

### [Step of producing compound (2-b)]

The compound represented by the formula (2-b) (hereinafter sometimes referred to as compound (2-b)) can be obtained in the form of a crystalline diastereomeric salt by treating compound (2) with (R)-α-phenylethylamine that is an optically active base in a solvent. Recrystallization of this (R)-α-phenylethylamine-added salt compound is repeatedly performed, so that compound (2-b) having a higher optical purity can be obtained.

Compound (2) and (R)-α-phenylethylamine are commercially available, but these compounds can also be synthesized by known methods.

The solvent in the salt resolution is not particularly limited. Preferred examples of the solvent include water, methanol, ethanol, isopropyl alcohol, diisopropyl ether, tetrahydrofuran, cyclopentyl methyl ether, dimethoxyethane, ethyl acetate, methylene chloride, chloroform, acetone, toluene, acetonitrile, and mixed solvents thereof. Among these, a mixed solvent of water and acetone (hereinafter sometimes referred to as aqueous acetone) and a mixed solvent of water and ethyl acetate (hereinafter sometimes referred to as aqueous ethyl acetate) are more preferable.

When aqueous acetone or aqueous ethyl acetate is used as a solvent in the salt resolution, the water content ratio is not particularly limited. It is preferably 0.1% to 3%, and more preferably 0.5% to 3%.

The amount of the solvent in the salt resolution is not particularly limited. The solvent is used in an amount (v/w) preferably 5 to 30 times, and more preferably 5 to 10 times higher than the amount of compound (2).

The crystallization temperature applied to the salt resolution differs depending on the solvent used. Crystallization can be carried out at a temperature from -10°C to the boiling point of the solvent. The crystallization temperature is preferably from 0°C to 60°C. The temperature may be kept constant. Alternatively, the temperature may be maintained for several hours at a temperature at which crystals are precipitated, and may be then be decreased stepwise. When the temperature is decreased stepwise, from the viewpoint of obtaining crystals having a high optical purity, it is preferable that the temperature be maintained, for example, at 40°C to 60°C for 2 to 6 hours, and then be gradually decreased (for example, at a rate of 5°C to 10°C/hour, and preferably at a rate of 5°C/hour, to a temperature of 20°C to 40°C, and then at a rate of 10°C/hour to a temperature of -10°C to 20°C).

The crystallization time applied to the salt resolution may be in the range from 1 hour to 48 hours, and is preferably in the range from 16 hours to 30 hours.

The amount of (R)-α-phenylethylamine added is not particularly limited. The (R)-α-phenylethylamine is used in an amount of, for example, 0.5 to 2 stoichiometric equivalents, and preferably 0.5 to 1 stoichiometric equivalent, based on compound (2).

The temperature at which the crystallized compound (2-b) is filtrated, is not particularly limited. It is preferably -20°C to 50°C, and more preferably -10°C to 30°C.

The precipitated crystal can be isolated, for example, by filtration, centrifugation, or a gradient method. The isolated crystal can be washed with a suitable solvent, as necessary.

Compound (2-b) obtained by optical resolution of compound (2) using the (R)-α-phenylethylamine salt is dissolved in a solvent by heating it, and it is then cooled for recrystallization, so that the optical purity of compound (2-b) can be further increased.

The solvent in the recrystallization is not particularly limited. Examples of preferred solvents include the same solvents as those used in production of the above described (R)-α-phenylethylamine-added salt compound. Aqueous acetone and aqueous ethyl acetate are preferable. When aqueous acetone or aqueous ethyl acetate is used, the water content ratio is not particularly limited. It is preferably 0.1% to 3%, and more preferably 0.5% to 3%. As a solvent in the recrystallization, a solvent different from the solvent used in the salt resolution may also be used. However, it is preferable to use the same solvent as that used in the salt resolution.

The amount of the solvent in the recrystallization is not particularly limited. The solvent is used in an amount (v/w) preferably 5 to 30 times, and more preferably 5 to 10 times higher than the amount of compound (2).

The crystallization temperature applied to the recrystallization differs depending on the solvent used. Crystallization can be carried out at a temperature from -10°C to the boiling point of the solvent, and the crystallization temperature is preferably 0°C to 60°C. The temperature may be kept constant. Alternatively, the temperature may be maintained for several hours at a temperature at which crystals are precipitated, and may then be decreased stepwise. When the temperature is decreased stepwise, from the viewpoint of obtaining crystals having a high optical purity, it is preferable that the temperature be maintained, for example, at 40°C to 60°C for 2 to 6 hours, and then be gradually decreased (for example, at a rate of 5°C to 10°C/hour, and preferably at a rate of 5°C/hour to a temperature of 20°C to 40°C, and then at a rate of 10°C/hour to a temperature of -10°C to 20°C) .

The crystallization time applied to the recrystallization may be in the range from 1 hour to 48 hours, and is preferably in the range from 16 hours to 30 hours.

The temperature at which compound (2-b) crystallized by recrystallization is filtrated, is not particularly limited. It is preferably -20°C to 50°C, and more preferably -10°C to 30°C.

The number of recrystallization operations is not particularly limited, as long as the compound of interest can be obtained with good optical purity and at a good yield. According to the method of the present invention, compound (2-b) having a high optical purity can be obtained by extremely few recrystallization operations, as few as five times or less, preferably three times or less, and more preferably two times or less. Accordingly, since the method of the present invention can be applied as an industrial production method of producing compound (2-b), further, compound (1 or 1-a) that is produced using compound (2-b), as described in detail below, and further, compound (X or X-a) useful as an activated blood coagulation factor X (FXa) inhibitor described in each of Patent Literatures 1 to 4, etc., the present method is extremely useful.

### [Step of producing compound (1)]

Compound (1) is produced by treating compound (2-b) with 1,3-dibromo-5,5-dimethylhydantoin (4) or N-bromosuccinimide (5) in a solvent, in the form of the salt, without converting it to the free acid. The present reaction is generally referred to as a bromolactonization reaction.

Herein, the 1,3-dibromo-5,5-dimethylhydantoin (4) and N-bromosuccinimide (5) that are used as bromine-supplying sources may be commercially available products.

The amount of the 1,3-dibromo-5,5-dimethylhydantoin (4) or N-bromosuccinimide (5) added is not particularly limited. These compounds can each be used in an amount of, for example, 0.5 to 2 equivalents, and preferably 0.6 to 1.2 equivalents, based on compound (2-b).

The reaction solvent in this step is not particularly limited, as long as it does not inhibit the reaction. Preferred examples of the reaction solvent include diethyl ether, diisopropyl ether, ethyl acetate, methylene chloride, chloroform, acetone, butanone, benzene, toluene, xylene, acetonitrile, and mixed solvents thereof. Acetonitrile is more preferable.

The amount of the solvent in this step is not particularly limited. The solvent is used in an amount (v/w) of preferably 1 to 20 times, and more preferably approximately 1.5 to 5 times higher than the amount of compound (2-b).

The reaction temperature applied to this step differs depending on the solvent used. This step can be carried out at a temperature from -10°C to the boiling point of the solvent. The temperature is preferably approximately 0°C to 40°C. The temperature may be kept constant. However, when the 1,3-dibromo-5,5-dimethylhydantoin (4) or N-bromosuccinimide (5) is added, the temperature is preferably decreased to approximately 0°C to 10°C.

As a post-treatment that is carried out after completion of the reaction, methods known as posttreatments of the halolactonization reaction may be applied. In general, sodium thiosulfate or an aqueous solution thereof, which is commonly used to capture excessive or free halogen (bromine atoms in the present reaction), is used. In addition, as an operation to separate a product of interest from the reaction system, such product of interest may be extracted using an organic solvent. The extraction solvent is not particularly limited. Examples of the extraction solvent that may be used herein include diethyl ether, diisopropyl ether, ethyl acetate, methylene chloride, chloroform, acetone, butanone, ethyl acetate, benzene, toluene, xylene, acetonitrile, and mixed solvents thereof.

Compound (2-b) as a starting substance in this step is a salt of amine. Thus, in order to remove unnecessary amine dissociated after completion of the reaction, it is preferable to wash the extract with an acid. The acid used herein may be either an organic acid or an inorganic acid. Examples of preferred acids include a hydrochloric acid aqueous solution, a sulfuric acid aqueous solution, an acetic acid aqueous solution, a tartaric acid aqueous solution, and a citric acid aqueous solution.

Compound (1) that is a product of this step can be isolated in the form of a solid (or a crystal) by filtration or the like.

As operations performed before the step such as filtration, concentration of extracts, completion of consolidation of the solid (or completion of crystallization of the crystal), washing, drying, and the like, can be carried out. The above described extract is concentrated, and a solvent preferable for precipitation of compound (1) is then added to the concentrate. Herein, an example of the solvent preferable for precipitation is isopropyl alcohol. The crystallization time may be in the range from 1 to 48 hours, and is preferably in the range of 16 to 30 hours. The crystallization temperature is 0°C or lower, and is preferably -5°C to -15°C.

The temperature at which the crystallized compound (1) is filtrated, is not particularly limited. It is preferably -5°C to -15°C.

The precipitated solid (or crystal) can be isolated, for example, by filtration, centrifugation, or a gradient method. The isolated crystal can be washed with a suitable solvent, as necessary. The washing solvent is preferably isopropyl alcohol.

From the (1S,4S,5S)-4-bromo-6-oxabicyclo[3.2.1]octan-7-one (1) produced in the present invention, edoxaban represented by the formula (X) and a p-toluenesulfonic acid monohydrate salt (X-a) of the formula (X) can be produced according to the production method described in International Publication No. WO 2003/000680. The specific steps will be described below.

Compounds (X and X-a) can be produced by the following [Step (a)] to [Step (e)].

Step (a): The (1S,4S,5S)-4-bromo-6-oxabicyclo[3.2.1]octan-7-one (1) represented by the formula (1) is treated with a dimethylamine aqueous solution to form a compound (5), then compound (5) is treated with an ammonia aqueous solution (ammonia water) to form a compound (7), then compound (7) is treated with di-tert-butyl dicarbonate to form a compound (8), and then compound (8) is treated with methanesulfonyl chloride to produce methanesulfonic acid (1R,2R,4S)-2-tert-butoxycarbonylamino-4-dimethylcarbamoyl-cyclohexyl ester represented by formula (9). 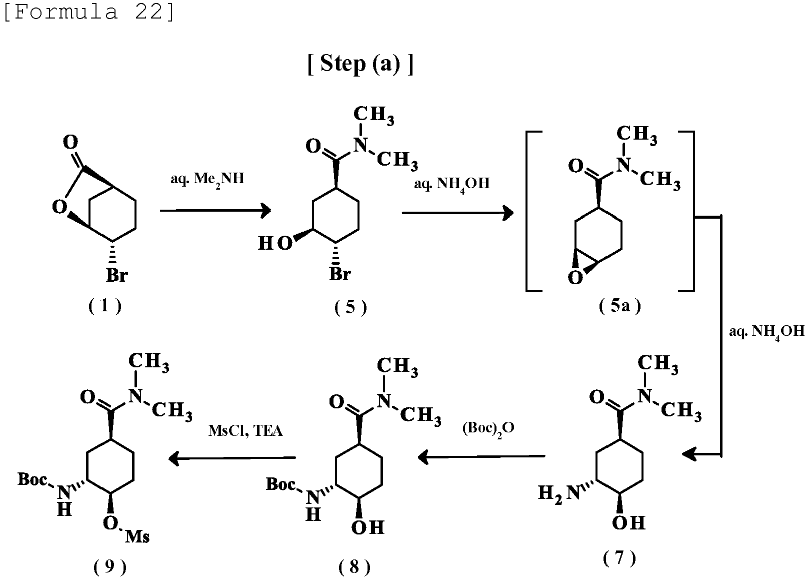 wherein Boc represents a tert-butoxycarbonyl group, and Ms represents a methanesulfonyl group.

Step (b): Compound (9) is treated with sodium azide to become a compound (10), then compound (10) is hydrogenated in the presence of palladium-carbon and ammonium formate to form a compound (11), and then compound (11) is treated with oxalic acid to produce tert-butyl {(1R,2S,5S)-2-amino-5-[(dimethylamino)carbonyl]cyclohexyl}carbamate oxalate represented by formula (11a). 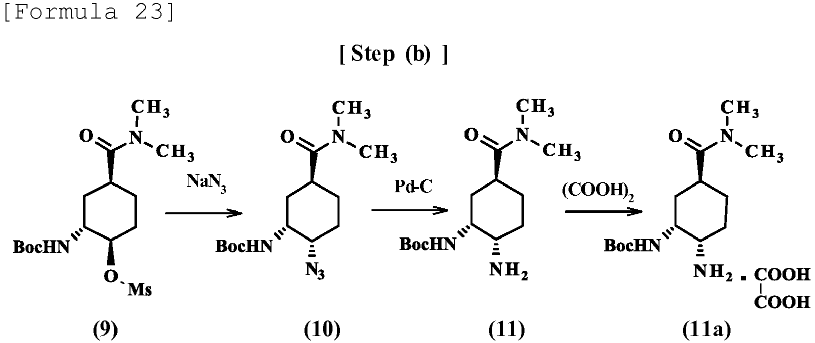 wherein Boc represents a tert-butoxycarbonyl group.

Step (c): Compound (11a) is treated with ethyl [5-chloropyridin-2-yl]amino](oxo)acetate hydrochloride in the presence of triethylamine to produce tert-butyl [[1R,2S,5S]-2-({[(5-chloropyridin-2-yl)amino](oxo)acetyl}amino)-5-(dimethylaminocarbonyl)cyclohexyl]carbamate represented by formula (12).

Step (d): The tert-butoxycarbonyl group of compound (12) is removed by treating it with methanesulfonic acid, and the resulting compound is then treated with 5-methyl-4,5,6,7-tetrahydro[1,3]thiazolo[5,4-c]pyridine-2-carboxylate hydrochloride in the presence of a condensing agent (water-soluble carbodiimide) to produce edoxaban represented by the formula (X). Thereafter, the subsequent [Step (e)] is carried out, as necessary. 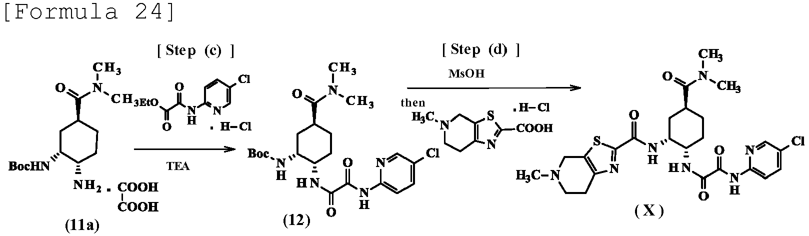 wherein Boc represents a tert-butoxycarbonyl group.

Step (e): Edoxaban represented by the formula (X), a pharmacologically acceptable salt thereof, or their hydrate is produced. For example, edoxaban represented by the formula (X) is treated with p-toluenesulfonic acid in aqueous ethanol to produce a p-toluenesulfonate monohydrate of compound (X), which is represented by the formula (X-a).

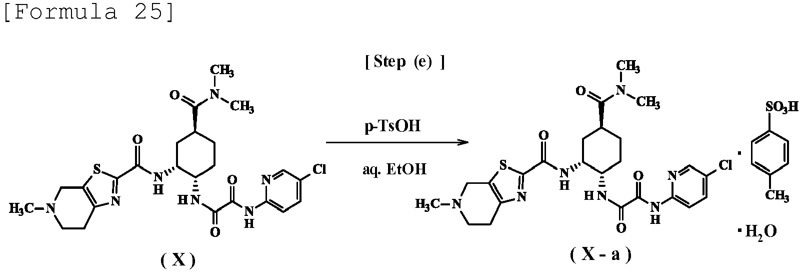

As described above, compound (1) is useful as an intermediate for the production of compounds (X and X-a) that are activated blood coagulation factor X (FXa) inhibitors, for example, described in Patent Literatures 2 to 5 and the like.

### Examples

Hereinafter, Examples will be described. However, these Examples are not intended to limit the present invention.

It is to be noted that the optical purity (%ee) of the obtained compound was obtained as follows.

### <Conditions for analysis of optical purity>

Detector: FID;
Column: J & W Cyclodex (registered trademark), 30 m x 0.25 mm;
Temperature in sample vaporizing chamber: 250°C;
Column temperature: 90°C;
Detection portion temperature: 250°C;
Carrier gas: helium;
Flow rate: 1 ml/min

### (Example 1)

### (1S,4S,5S)-4-Bromo-6-oxabicyclo[3.2.1]octan-7-one (1)

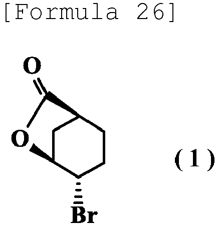

Under a nitrogen atmosphere, 1,3-dibromo-5,5-dimethylhydantoin (4) (11.6 g, 40.6 mol) was added to a solution of (S)-3-cyclohexene-1-carboxylic acid (R)-α-phenylethylamine salt (2-b) (10.0 g, 40.4 mmol) in acetonitrile (25 ml) that had been cooled on ice. The temperature of the reaction solution was increased to room temperature, and the reaction solution was then stirred for 2 hours. Ethyl acetate (50 ml) and a 10% sodium thiosulfate aqueous solution (40 ml) were added to the reaction solution, and the organic layer was separated. The aqueous layer was extracted with ethyl acetate (50 ml), and the organic layers were gathered and were then washed with a 5% citric acid aqueous solution (40 ml). The resulting organic layer was washed with a saturated saline (30 ml) and was then concentrated under reduced pressure. Isopropyl alcohol (50 ml) was added to the residue, and the solvent was then distilled off under reduced pressure, so that the volume was concentrated to 30 ml. The obtained slurry product was cooled to -10°C, and a precipitate was then filtrated and was washed with ice-cooled isopropyl alcohol (10 ml) to obtain the title compound (1) (6.9 g, yield: 83.5%).

Various spectrum data of compound (1) were matched with the data described in the publication (Non-patent Literature 4).

### (Example 2)

### {(1R,2R,4S)-2-[(tert-Butoxycarbonyl)amino]-4-[(dimethylamino)carbonyl]cyclohexyl}methanesulfonate (9)

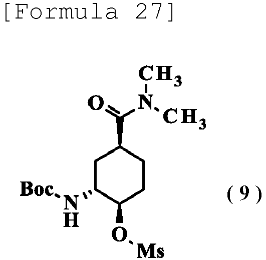

wherein Boc represents a tert-butoxycarbonyl group, and Ms represents a methanesulfonyl group.

(1S,4S,5S)-4-Bromo-6-oxabicyclo[3.2.1]octan-7-one (20 g) was dissolved in acetonitrile (125 ml), and a 50% dimethylamine aqueous solution (35.2 g) was then added to the obtained solution at approximately 10°C. The reaction mixture was stirred at 10°C for 15 hours, and the reaction solvent was then distilled off at 15°C or lower under reduced pressure. 28% ammonia water (125 ml) was added to the residue. The mixed solution was heated to 35°C to 45°C, and it was stirred for 8 hours. Then, the reaction solution was further stirred at 25°C for 14 hours. The reaction solvent was distilled off under reduced pressure. Deionized water (63 ml) was added to the residue, and the obtained solution was then concentrated under reduced pressure again. Then, deionized water (88 ml), di-tert-butyl dicarbonate (31.9 g), and a 48% sodium hydroxide aqueous solution (20.3 g) were added to the concentrated residue, and the mixed solution was stirred at 40°C to 45°C for 2 hours. 4-Methyl-2-pentanone (175 ml) was added to the reaction mixture, and the organic layer was separated. The aqueous layer was extracted with 4-methyl-2-pentanone (175 ml). Extracts were gathered, and the solvent was concentrated under reduced pressure until the total volume became 175 ml. 4-Methyl-2-pentanone (100 ml) was added to the concentrated residue, and the solvent was concentrated under reduced pressure until the total volume of the mixed solution became 175 ml. 4-Methyl-2-pentanone was added to the concentrated residue to a total volume of 250 ml. Methanesulfonyl chloride (17.9 g) was added to the mixed solution, and then, triethylamine (18.8 g) was gradually added to the mixed solution such that the temperature of the mixed solution was kept at 15°C to 30°C. The reaction mixed solution was stirred at the same temperature as described above for 1 hour. Completion of the reaction was confirmed, and methanol (43 ml) and deionized water (63 ml) were then added to the reaction mixed solution, followed by stirring the mixture for 15 minutes. The organic layer was separated and was washed with a 5% sodium hydrogen carbonate aqueous solution (50 ml). The organic layer was then concentrated under reduced pressure, so that the total volume became 100 ml. The obtained slurry product was stirred at approximately 0°C for 3 hours. A precipitate was filtrated, and was washed with 4-methyl-2-pentanone (25 ml), followed by drying under reduced pressure, to obtain the title compound (9) (22.4 g, yield: 62. 9%).
HPLC purity: 99.23%

### (Reference Example 1)

### (S)-3-Cyclohexene-1-carboxylic acid (R)-α-phenylethylamine salt (2-b)

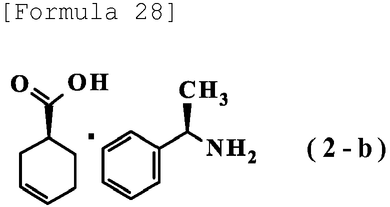

3-Cyclohexene-1-carboxylic acid (1) (1.0 kg) was dissolved in 4.8% aqueous acetone (7.5 1), and, a solution (500 ml) prepared by dissolving (R)-α-phenylethylamine (3) (624.3 g) in 4.8% aqueous acetone was gradually added to the above obtained solution at 50°C. The obtained mixture was stirred at the same temperature as described above for 4 hours. The suspension was cooled to 35°C, and it was stirred at the same temperature as described above for 16 hours. Then, the suspension was further stirred at 10°C for 3 hours. The suspension was filtrated under reduced pressure, so that 837.1 g of the title salt compound was obtained in the form of a crystal. The optical purity thereof was 63%de. Subsequently, 4.8% aqueous acetone (5.6 1) was added to 700 g of the obtained salt compound, and the obtained mixture was stirred under heating to reflux for 5 hours, and at 30°C for 13 hours. After that, the reaction mixture was stirred under cooling on ice for 3 hours. The suspension was filtrated under reduced pressure to obtain 519.4 g of the title salt compound in the form of a crystal. The optical purity was 81%de. Further, 4.8% aqueous acetone (4.0 1) was added to 500 g of the obtained salt compound, and the obtained mixture was stirred under heating to reflux for 5 hours, and at 30°C for 13 hours, and after that, the reaction mixture was stirred at 10°C for 3 hours. The suspension was filtrated under reduced pressure to obtain 398.5 g of the title salt compound in the form of a crystal. The optical purity thereof was 91%de. Finally, 4.8% aqueous acetone (2.4 1) was added to 300 g of the obtained salt compound, and the obtained mixture was stirred under heating to reflux for 5 hours, and at 30°C for 13 hours, and after that, the reaction mixture was stirred at 10°C for 3 hours. The suspension was filtrated under reduced pressure to obtain 240.0 g of the title compound (2-b) in the form of a crystal. The optical purity thereof was 97%de.

¹H-NMR (D₂O) δ: 1.50-1.63 (1H, m), 1.66 (3H, d, J = 6.9 hz), 1.86-1.95 (1H, m), 1.98-2.25 (4H, m), 2.32-2.43 (1H, m), 4.56 (1H, q, J = 6.9 Hz), 5.70-5.80 (2H, m), 7.40-7.55 (5H, m).
Anal: C₁₅H₂₁NO₂
Theoretical (%) C; 72.84, H; 8.56, N; 5.66.
Found (%) C; 72.88, H; 8.58, N; 5.72.

### (Reference Example 2)

### (R)-α-Phenylethylamine salt of (S)-3-cyclohexene-1-carboxylic acid (2-b)

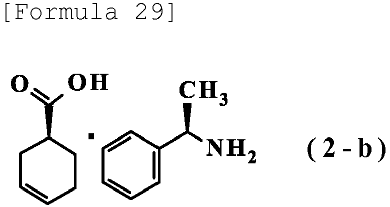

3-Cyclohexene-1-carboxylic acid (1) (30 g) was dissolved in 3% aqueous ethyl acetate (150 ml), and a solution (30 ml) prepared by dissolving (R)-α-phenylethylamine (3) (23.0 g) in 3% aqueous ethyl acetate was gradually added to the obtained solution at 55°C. The obtained mixture was stirred at the same temperature as described above for 6 hours. The suspension was stirred at 25°C for 5 hours, and further at -10°C for 2 hours and 30 minutes. The suspension was filtrated under reduced pressure to obtain 32.9 g of the title salt compound in the form of a crystal. The optical purity thereof was 49%de. Subsequently, 3% aqueous ethyl acetate (196 ml) was added to 32.7 g of the obtained salt compound, and the obtained mixture was stirred at 55°C for 3 hours, then at 25°C for 5 hours, and further at - 10°C for 2 hours and 30 minutes. The suspension was filtrated under reduced pressure to obtain 24.7 g of the title salt compound in the form of a crystal. The optical purity thereof was 78%de. Further, 3% aqueous ethyl acetate (148 ml) was added to 24.6 g of the obtained salt compound, and the obtained mixture was stirred at 55°C for 3 hours, then at 25°C for 5 hours, and further at-10°C for 2 hours and 30 minutes. The suspension was filtrated under reduced pressure to obtain 20.3 g of the title compound (2-b) in the form of a crystal. The optical purity thereof was 95%de.

### (Reference Example 3)

### tert-Butyl {(1R,2S,5S)-2-amino-5-[(dimethylamino)carbonyl]cyclohexyl}carbamate oxalate (11a)

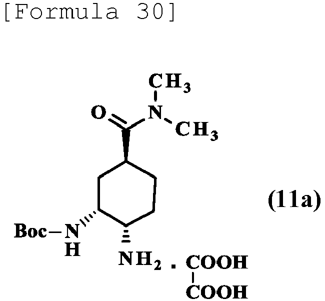

wherein Boc represents a tert-butoxycarbonyl group.

Sodium azide (7.14 g) and dodecyl pyridinium chloride (7.80 g) were added to a solution of methanesulfonic acid (1R,2R,4S)-2-tert-butoxycarbonylamino-4-dimethylcarbamoyl-cyclohexyl ester (20.0 g) in toluene (100 mL) at room temperature, and the obtained mixture was stirred at 60°C for 72 hours. Water was added to the reaction solution, and the organic layer was separated. The organic layer was washed with a saturated sodium hydrogen carbonate aqueous solution and water. To the thus washed organic layer, methanol, 7.5% palladium-carbon, and ammonium formate were added, and the obtained mixture was stirred at 40°C for 1 hour. The palladium-carbon was removed by filtration, and the solvent was distilled off under reduced pressure. Aqueous acetonitrile (200 mL) and anhydrous oxalic acid (4.94 g) were added to the residue. The mixture was stirred at room temperature for 17 hours, and a precipitated crystal was filtrated. Acetonitrile (200 mL) was added to the crystal that had been collected by filtration, and the obtained mixture was stirred at 40°C for 24 hours. A precipitated crystal was filtrated and was dried to obtain 12.7 g of the title product.

### (Reference Example 4)

### tert-Butyl [(1R,2S,5S)-2-({[(5-chloropyridin-2-yl)amino](oxo)acetyl}amino)-5-(dimethylaminocarbonyl)cyclohexyl]carbamate (12)

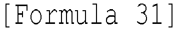

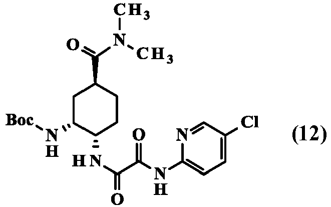

tert-Butyl {(1R,2S,5S)-2-amino-5-[(dimethylamino)carbonyl]cyclohexyl}carbamate oxalate (11a) (100.1 g) was suspended in acetonitrile (550 mL), and triethylamine (169 mL) was added to the suspension at 60°C. Ethyl [5-chloropyridin-2-yl]amino](oxo)acetate hydrochloride (84.2 g) was added to the mixture at the same temperature as described above, and the thus obtained mixture was stirred for 6 hours. Thereafter, the reaction mixture was cooled to room temperature, and it was stirred for 16 hours. Water was added to the reaction mixture, and the obtained mixture was stirred at 10°C for 1.5 hours. A precipitated crystal was filtrated and was dried to obtain 106.6 g of the title product.

### (Reference Example 5)

### N¹-(5-Chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide [edoxaban] (X)

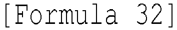

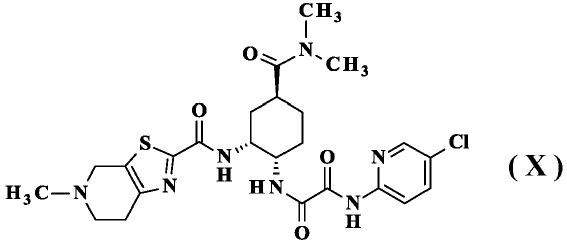

tert-Butyl [(1R,2S,5S)-2-({[(5-chloropyridin-2-yl)amino](oxo)acetyl}amino)-5-(dimethylaminocarbonyl)cyclohexyl]carbamate (12) (95.1 g) was suspended in acetonitrile (1900 mL), and methanesulfonic acid (66 mL) was added to the suspension at room temperature. The obtained mixture was stirred at the same temperature as described above for 2 hours. While the reaction mixture was stirred under cooling on ice, triethylamine (155 mL), 5-methyl-4,5,6,7-tetrahydro[1,3]thiazolo[5,4-c]pyridine-2-carboxylate hydrochloride (52.5 g), 1-hydroxybenzotriazole (33.0 g), and 1-ethyl-3-(3-dimethylaminopropyl)carbodiimide hydrochloride (46.8 g) were added to the mixture. The reaction mixture was stirred at room temperature for 16 hours. Triethylamine and water were added to the reaction mixture, and the obtained mixture was then stirred under cooling on ice for 1 hour. A precipitated crystal was collected by filtration and was dried to obtain 103.2 g of the title product.

### (Reference Example 6)

### Synthesis of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (X-a) (the method described in International Publication No. WO 07/032498)

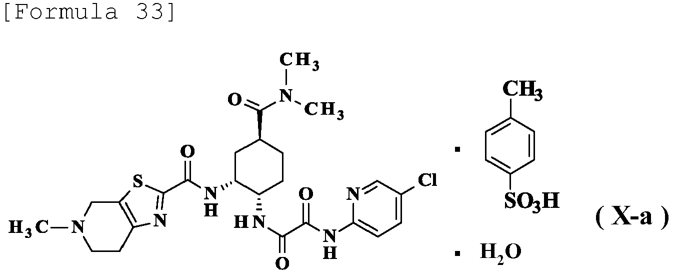

N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide (86.8 g) was dissolved in 30% aqueous ethanol (418 ml) at 60°C, and a solution of p-toluenesulfonic acid monohydrate (29.0 g) in 30% aqueous ethanol (167 ml) was then added to the above obtained solution. The reaction mixture was stirred at 70°C for 1 hour, and thereafter, it was gradually cooled to room temperature. After that, ethanol was added to the mixture, and the obtained mixture was stirred for 16 hours. The reaction solution was stirred under cooling on ice for 1 hour, and a crystal was collected by filtration to obtain 102.9 g of the title compound.

### Industrial Applicability

The present invention can be utilized for the production of compound (X) and compound (X-a) that are FXa inhibitors.

## Claims

1. A method for producing a compound represented by the following formula (1) : 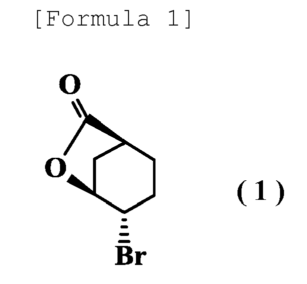 wherein the method comprises treating an (R)-α-phenylethylamine salt of (S)-3-cyclohexene-1-carboxylic acid represented by the following formula (2-b): 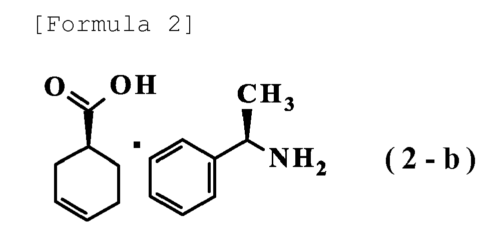 with 1,3-dibromo-5,5-dimethylhydantoin represented by the following formula (4): 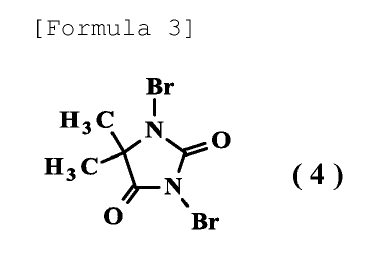 or with N-bromosuccinimide represented by the following formula (5): 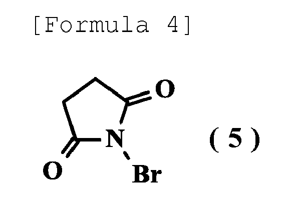 in a solvent.

2. A method for producing a compound represented by the following formula (1) : 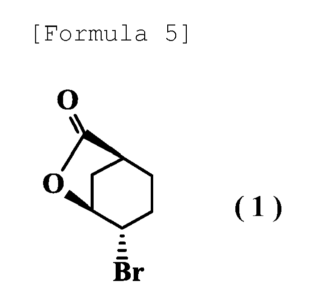 wherein the method comprises treating 3-cyclohexene-1-carboxylic acid represented by the following formula (2) : 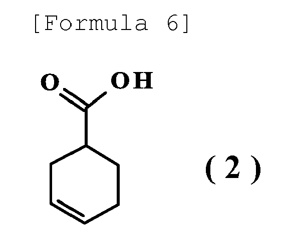 with (R)-α-phenylethylamine represented by the following formula (3): 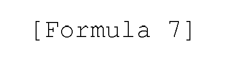 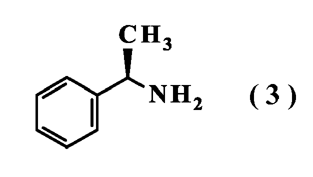 to obtain an (R)-α-phenylethylamine salt of (S)-3-cyclohexene-1-carboxylic acid represented by the following formula (2-b): 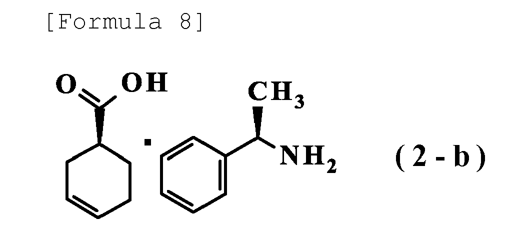 and then treating the (R)-α-phenylethylamine salt of (S)-3-cyclohexene-1-carboxylic acid with 1,3-dibromo-5,5-dimethylhydantoin represented by the following formula (4) : 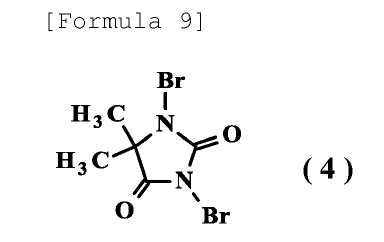 or with N-bromosuccinimide represented by the following formula (5): 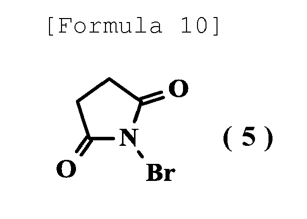 in a solvent.

3. The production method according to claim 1 or 2, wherein the solvent is acetonitrile.

4. A method for producing a compound represented by the following formula (11a) : 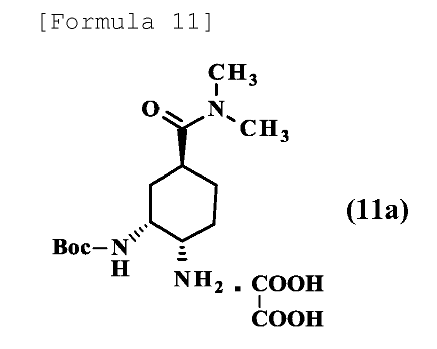 wherein Boc represents a tert-butoxycarbonyl group;
wherein (1S,4S,5S)-4-bromo-6-oxabicyclo[3.2.1]octan-7-one (1) represented by the formula (1), which is produced by a production method according to claim 1, is used, and the method comprises the following steps (a) and (b):
step (a): treating the (1S,4S,5S)-4-bromo-6-oxabicyclo[3.2.1]octan-7-one (1) with a dimethylamine aqueous solution, then treating the resulting compound with an ammonia aqueous solution, then treating the resulting compound with di-tert-butyl dicarbonate, and then treating the resulting compound with methanesulfonyl chloride to produce methanesulfonic acid (1R,2R,4S)-2-tert-butoxycarbonylamino-4-dimethylcarbamoyl-cyclohexyl ester; and
step (b): treating the methanesulfonic acid (1R,2R,4S)-2-tert-butoxycarbonylamino-4-dimethylcarbamoyl-cyclohexyl ester with sodium azide, then hydrogenating the resulting compound in the presence of palladium-carbon and ammonium formate, and then treating the resulting compound with oxalic acid to produce tert-butyl {(1R,2S,5S)-2-amino-5-[(dimethylamino)carbonyl]cyclohexyl}carbamate oxalate (11a).

5. A method for producing N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide p-toluenesulfonate monohydrate represented by the following formula (X-a): 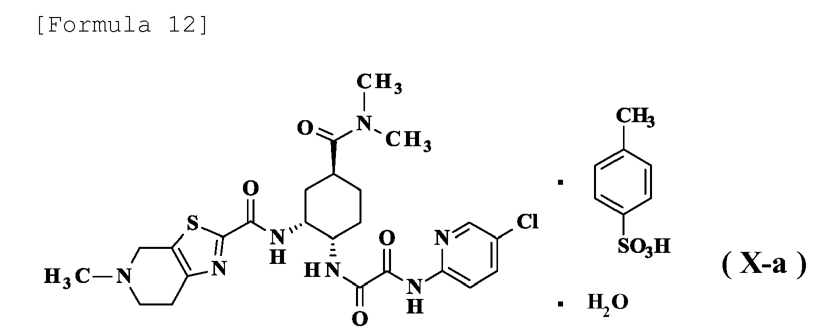 wherein (1S,4S,5S)-4-bromo-6-oxabicyclo[3.2.1]octan-7-one (1) represented by the formula (1), which is produced by a production method according to claim 1, is used, and the method comprises the following steps (a) to (e) :
step (a): treating the (1S,4S,5S)-4-bromo-6-oxabicyclo[3.2.1]octan-7-one (1) with a dimethylamine aqueous solution, then treating the resulting compound with an ammonia aqueous solution, then treating the resulting compound with di-tert-butyl dicarbonate, and then treating the resulting compound with methanesulfonyl chloride to produce methanesulfonic acid (1R,2R,4S)-2-tert-butoxycarbonylamino-4-dimethylcarbamoyl-cyclohexyl ester;
step (b): treating the methanesulfonic acid (1R,2R,4S)-2-tert-butoxycarbonylamino-4-dimethylcarbamoyl-cyclohexyl ester with sodium azide, then hydrogenating the resulting compound in the presence of palladium-carbon and ammonium formate, and then treating the resulting compound with oxalic acid to produce tert-butyl {(1R,2S,5S)-2-amino-5-[(dimethylamino)carbonyl]cyclohexyl}carbamate oxalate;
step (c): treating the tert-butyl {(1R,2S,5S)-2-amino-5-[(dimethylamino)carbonyl]cyclohexyl}carbamate oxalate with ethyl [5-chloropyridin-2-yl]amino](oxo)acetate hydrochloride in the presence of triethylamine to produce tert-butyl [[1R,2S,5S]-2-({[(5-chloropyridin-2-yl)amino](oxo)acetyl}amino)-5-(dimethylaminocarbonyl)cyclohexyl]carbamate;
step (d): treating the tert-butyl [(1R,2S,5S)-2-({[(5-chloropyridin-2-yl)amino](oxo)acetyl}amino)-5-(dimethylaminocarbonyl)cyclohexyl]carbamate with methanesulfonic acid, and then treating the resulting compound with 5-methyl-4,5,6,7-tetrahydro[1,3]thiazolo[5,4-c]pyridine-2-carboxylate hydrochloride to produce N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide [edoxaban]; and
step (e): treating the N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide [edoxaban] with p-toluenesulfonic acid in aqueous ethanol to produce N¹-(5-chloropyridin-2-yl)-N²-[(1S,2R,4S)-4-(dimethylcarbamoyl)-2-{[(5-methyl-4,5,6,7-tetrahydro[1,3]thiazolo[5,4-c]pyridin-2-yl)carbonyl]amino}cyclohexyl]ethanediamide p-toluenesulfonate monohydrate (X-a).
